# EUROPEAN PATENT SPECIFICATION

(11) **EP 3 609 323 B1**
(45) Date of publication and mention of the grant of the patent: **13.11.2024**
(21) Application number: 18767995.6
(22) Date of filing: 08.03.2018
(51) Int. Cl.: A01N 1/02, A01N 3/00, G01N 33/48, C12N 15/10, C12Q 1/6806

(54) **METHODS AND COMPOSITIONS FOR PRESERVING TISSUES AND NUCLEIC ACIDS**
VERFAHREN UND ZUSAMMENSETZUNGEN ZUR KONSERVIERUNG VON GEWEBEN UND NUKLEINSÄUREN
PROCÉDÉS ET COMPOSITIONS DE PRÉSERVATION DE TISSUS ET D'ACIDES NUCLÉIQUES

(30) Priority: 17.03.2017 US 201762472828 P
(43) Date of publication of application: 19.02.2020
(73) Proprietor: Syngenta Crop Protection AG, 4058 Basel (CH)
(72) Inventor: JI, Yanshan, RTP, North Carolina 27709 (US)
(74) Representative: SYNGENTA IP
(86) International application number: PCT/US2018/021512
(87) International publication number: WO 2018/169754

(56) References cited:
- EP-A1- 1 804 045
- EP-B1- 1 804 045
- WO-A1-2011/109031
- WO-A1-2014/018195
- WO-A1-2014/018195
- US-A- 5 500 339
- CHIA-YING CHIANG ET AL: "Carbon dioxide capture by sodium hydroxide-glycerol aqueous solution in a rotating packed bed", JOURNAL OF THE TAIWAN INSTITUTE OF CHEMICAL ENGINEERS, vol. 72, 6 February 2017 (2017-02-06), AMSTERDAM, NL, pages 29 - 36, XP055729769, ISSN: 1876-1070, DOI: 10.1016/j.jtice.2017.01.023
- SCHAUDIEN D ET AL: "High Preservation of DNA standards Diluted in 50% Glycerol", DIAGNOSTIC MOLECULAR PATHOLOGY, LIPPINCOTT WILLIAMS AND WILKINS, US, vol. 16, no. 3, 1 January 2007 (2007-01-01), pages 153 - 157, XP009517867, ISSN: 1052-9551, DOI: 10.1097/PDM.0B013E31803C558A

## Description

### FIELD OF THE INVENTION

The presently disclosed subject matter relates to methods, compositions, and kits for preserving and preparing tissue samples containing nucleic acids. The present invention relates generally to reagents and methods capable for simple and effective preservation and processing of a large number of biological samples. More specifically, the invention relates to reagents and methods for storing and/or processing of biological samples for direct use in PCR and other DNA applications. The present invention further provides for reagents and methods to process biological samples and make DNA usable in PCR without further DNA purification. The presently disclosed subject matter can also be employed in low throughput and quick point-of-care diagnostic applications.

### BACKGROUND

Polymerase chain reaction (PCR) in molecular diagnostics has made it desirable to develop reagents and methods capable of simple and effective preservation and processing of a large number of biological samples.

Traditional alkaline lysis requires the following steps: concentrating or pelleting cells diluted in growth media; centrifuging or vortexing; lysing cells with alkaline detergent; shaking and/or agitating lysate; adding neutralization buffer; filtering and/or manipulating sample to remove the flocculent mass; adding a solid phase carrier; and adding binding buffer. Additional purification steps are generally required to remove the detergents and salts as follows: addition of solid phase carriers and addition of a binding buffer.

A frequently used source in DNA diagnostic is blood although buccal swabs and tissue biopsies are also often used, especially for the cancer testing. Currently, tissue samples are stored and preserved frozen, formalin-fixed or paraffin-imbedded. The use of formalin-fixed or paraffin-imbedded samples require a multi-step process to render DNA from these samples usable for PCR. Blood storage for DNA diagnostic purposes requires refrigeration, freeze-drying or freezing. Alternatively, blood samples are applied to a card, such as an FTA CARD (Invitrogen) and store dry at room temperature. Leal-Klevez et al. developed a method for storing blood and tissue samples at -20 C in 20% ethylene glycol-propylene glycol aqueous solution. In this protocol, DNA can be extracted from the stored sample by the proteinase K-phenol extraction procedure. Regarding the preservation of DNA samples for extended periods, Schaudien et al., 2007 (Diagnostic Molecular Pathology vol. 16, no. 3), discloses that the use of 50% glycerol may be used to improve the survival of DNA samples to repeated freeze-thawed cycles.

EP 1804045 describes a procedure for preserving a tissue sample whereby a biological sample is obtained and contacted with a composition comprising at least a polyol so as to store the sample at different temperatures.

U.S. Pat. No. 6,602,718 describes methods and reagents for stabilizing RNA and DNA in biological samples using an aqueous solution or dispersion of cationic detergent supplemented with additives including chaotropic salts, at a pH range from about 2 to about 12.

Various methods of sample processing have been used to prepare DNA for the PCR analysis. DNA is typically extracted using phenol-based methods, salt-alcohol precipitation method or adsorption on column methods. A review of these methods is provided in Current Protocols pp. 2.0.1-2.4.5. Also, U.S. Pat. Nos. 5,346,994 and 5,945,515 described reagents and methods for DNA isolation. These reagents and methods provide substantially pure DNA. However, these are time consuming and not easy adaptable for automation. Methods for performing PCR on samples without prior isolation of DNA have been developed such as: heating samples at 94° C. (Mercier et al.), microwave irradiation (Ohhara et al.) and treatment with thermophilic protease (Belly et al.). Many of these methods relay on 40 cycle-PCR amplification. A 40-cycle PCR amplification is prone to artifacts and cannot be reliably used for diagnostic purposes.

Previously, alkaline lysis was used to prepare blood and other samples directly for PCR, without purification of DNA. Rudbeck et al. used 0.2 M NaOH to lyse at room temperature whole blood, semen and epithelial cells. The lysate was neutralized and used directly for PCR 20 mM NaOH was not effective in releasing DNA usable for PCR. Ashen et al. modified this protocol by first isolating blood leukocytes by centrifugation and lysing. The isolated leukocytes were lysed in 0.2 M NaOH at room temperature. The lysate was neutralized and used for PCR.

Truett et al. used 25 mM NaOH-0.2 mM disodium EDTA buffer, pH 12 to lyse mouse tissue samples by heating at 95 C. After heating, sample lysates were neutralized and used for PCR. This method was not very effective since it involved 40 cycles of PCR to amplify DNA fragments. Also, neutralization of the alkaline lysate added one step to the protocol and made strict requirements for accurate determination of pH in the lysate.

Lin et al. described preparation of DNA from blood by first removing hemoglobin from the whole blood by clotting the blood for a minimum 2 h followed by overnight storage and centrifugation. Resulted serum was subjected to lysis in 18 mM KOH at 37 C, neutralized and used for PCR.

Rapid protocols for DNA analysis by PCR were also detailed for bacteria. Sandhu et al. boiled bacteria colonies in water. Joshi et al. used bacteria directly for PCR without boiling. An FTA CARD method for storing and processing of bacterial cells were also reported (Rogers et al.).

Sampling plant tissues and preserving those tissues and the nucleic acids they contain had proven more difficult than blood or other cellular tissues in many respects. Tissue sampling from plants is typically done in the field or in greenhouses-places where conditions are usually hot and quite varied. Until now, sampling was very labor-intensive and slow, often requiring samples be kept on dry ice until they could properly be stored.

A method for isolating nucleic acids from plant material using a lysis buffer comprising tromethamine, sodium chloride, EDTA, sodium dodecylsulfate, 125 mM NaOH-, 1 vol. % glycerol and 1 vol. % polyvinyl pyrrolidone was described in WO 2014/018195. This method required separating the liquid and solid phase and recovering the nucleic acid from the liquid phase. However, preservation of DNA was not shown/possible for a long period of time (not more than 30 min) at room temperature for posterior use of the DNA, e.g., for PCR. Thus, there is now a need for a reliable and shelfstable reagents and methods for the ambient temperature storage and which are compatible with reagents and methods for processing biological samples for direct PCR. Unexpectedly, it has now been found that reagents comprising of glycerol preserve DNA and secure long-term ambient temperature storage of plant samples, even while the alkaline aspect of the reagent lyses cells of the plant samples.

### SUMMARY

The invention is a method of preserving a tissue sample containing nucleic acids, which is then used for DNA analysis. In one embodiment, the tissue is plant tissue, preferably at least one leaf disc. Once obtained, the tissue sample is placed in contact with an alkaline glycerol reagent. This reagent allows for storage of the tissue sample at room temperatures (that is, cold or frozen storage is not required) without significant loss of DNA quality The reagent comprises an amount of alkali, glycerol and a detergent, wherein the alkali is capable of maintaining the pH of the reagent from pH 12 to pH 13.8, wherein the glycerol is at a concentration of from 10% to 25%, and wherein the detergent is polyvinyl pyrrolidone (PVP). Preferably, the alkali is sodium hydroxide. Preferably, the glycerol is at a concentration of 15% or 20%.

The invention is also a kit for tissue sampling. The kit may include a vessel, such as an Eppendorf tube or a series of tubes or a 96-well block, and the reagent of the invention (which may be pre-loaded into the tubes or wells).

The invention is also a method for extracting nucleic acids from tissue samples. The method comprises: a. obtaining a tissue sample containing nucleic acids; b. contacting the tissue sample with the reagent according to the invention (defined in claim 2); and c. disrupting the tissue sample. Disruption can occur by various means, for example, mortar and pestle, or grinding in a mill or with a steel bead. Once the nucleic acids are released into the reagent, the glycerol in the reagent allows for the preservation of the nucleic acids, or the nucleic acids may be used immediately for analysis.

### DEFINITIONS

The invention is as defined in the appended set of claims.

It is also to be understood that the terminology used herein is for the purpose of describing particular embodiments only, and is not intended to limit the scope of the present invention, which will be limited only by the appended claims. It must be noted that as used herein and in the appended claims, the singular forms "a," "and," and "the" include plural reference unless the context clearly dictates otherwise. Thus, for example, reference to "a plant" is a reference to one or more plants and includes equivalents thereof known to those skilled in the art, and so forth.

The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent, preferably 10 percent up or down (higher or lower). With regard to a temperature the term "about" means ± 1 °C, preferably ± 0.5°C. Where the term "about" is used in the context of this invention (e.g., in combinations with temperature or molecular weight values) the exact value (i.e., without "about") is preferred.

As used herein, the term "amplified" means the construction of multiple copies of a nucleic acid molecule or multiple copies complementary to the nucleic acid molecule using at least one of the nucleic acid molecules as a template. Amplification systems include the polymerase chain reaction (PCR) system, ligase chain reaction (LCR) system, nucleic acid sequence based amplification (NASBA, Cangene, Mississauga, Ontario), Q-Beta Replicase systems, transcription-based amplification system (TAS), and strand displacement amplification (SDA). See, e.g., Diagnostic Molecular Microbiology: Principles and Applications, PERSING et al., Ed., American Society for Microbiology, Washington, D.C. (1993). The product of amplification is termed an "amplicon."

The term "specific DNA sequence" indicates a polynucleotide sequence having a nucleotide sequence homology of more than 80%, preferably more than 85%, more preferably more than 90%, even more preferably more than 95%, still more preferably more than 97%, most preferably more than 99% with another named sequence.

"cDNA" refers to a single-stranded or a double-stranded DNA that is complementary to and derived from mRNA.

The term "chimeric construct", "chimeric gene", "chimeric polynucleotide" or chimeric nucleic acid" (and similar terms) as used herein refers to a construct or molecule comprising two or more polynucleotides of different origin assembled into a single nucleic acid molecule. The term "chimeric construct", "chimeric gene", "chimeric polynucleotide" or "chimeric nucleic acid" refers to any construct or molecule that contains (1) polynucleotides (e.g., DNA) , including regulatory and coding polynucleotides that are not found together in nature (i.e., at least one of polynucleotides is heterologous with respect to at least one of its other polynucleotides), or (2) polynucleotides encoding parts of proteins not naturally adjoined, or (3) parts of promoters that are not naturally adjoined. Further, a chimeric construct, chimeric gene, chimeric polynucleotide or chimeric nucleic acid may comprise regulatory polynucleotides and coding polynucleotides that are derived from different sources, or comprise regulatory polynucleotides and coding polynucleotides derived from the same source, but arranged in a manner different from that found in nature. In a preferred aspect of the present invention the chimeric construct, chimeric gene, chimeric polynucleotide or chimeric nucleic acid comprises an expression cassette comprising a polynucleotides of the present invention under the control of regulatory polynucleotides, particularly under the control of regulatory polynucleotides functional in plants.

The term "chromosome" is used herein as recognized in the art as meaning the self-replicating genetic structure in the cellular nucleus containing the cellular DNA and bearing the linear array of genes.

A "coding polynucleotide" is a polynucleotide that is transcribed into RNA, such as mRNA, rRNA, tRNA, snRNA, sense RNA or antisense RNA. Preferably the RNA is then translated in an organism to produce a protein. It may constitute an "uninterrupted coding polynucleotide", i.e., lacking an intron, such as in a cDNA, or it may include one or more introns bounded by appropriate splice junctions. An "intron" is a poly(ribo)nucleotide which is contained in the primary transcript but which is removed through cleavage and religation of the RNA within the cell to create the mature mRNA that can be translated into a protein.

"dsRNA" or "double-stranded RNA" is RNA with two substantially complementary strands, which directs the sequence-specific degradation of mRNA through a process known as RNA interference (RNAi). dsRNA is cut into siRNAs interfering with the expression of a specific gene.

The term "expression" when used with reference to a polynucleotide, such as a gene, ORF or portion thereof, or a transgene in plants, refers to the process of converting genetic information encoded in a gene into RNA (e.g., mRNA, rRNA, tRNA, or snRNA) through "transcription" of the gene (i.e., via the enzymatic action of an RNA polymerase), and into protein where applicable (e.g. if a gene encodes a protein), through "translation" of mRNA. Gene expression can be regulated at many stages in the process. For example, in the case of antisense or dsRNA constructs, respectively, expression may refer to the transcription of the antisense RNA only or the dsRNA only. In embodiments, "expression" refers to the transcription and stable accumulation of sense (mRNA) or functional RNA. "Expression" may also refer to the production of protein.

"Expression cassette" as used herein means a nucleic acid molecule capable of directing expression of a particular polynucleotide or polynucleotides in an appropriate host cell, comprising a promoter operably linked to the polynucleotide or polynucleotides of interest which is/are operably linked to termination signals. It also typically comprises polynucleotides required for proper translation of the polynucleotide or polynucleotides of interest. The expression cassette may also comprise polynucleotides not necessary in the direct expression of a polynucleotide of interest but which are present due to convenient restriction sites for removal of the cassette from an expression vector. The expression cassette comprising the polynucleotide(s) of interest may be chimeric, meaning that at least one of its components is heterologous with respect to at least one of its other components. The expression cassette may also be one that is naturally occurring but has been obtained in a recombinant form useful for heterologous expression. Typically, however, the expression cassette is heterologous with respect to the host, i.e. the particular polynucleotide of the expression cassette does not occur naturally in the host cell and must have been introduced into the host cell or an ancestor of the host cell by a transformation process known in the art. The expression of the polynucleotide(s) in the expression cassette is generally under the control of a promoter. In the case of a multicellular organism, such as a plant, the promoter can also be specific or preferential to a particular tissue, or organ, or stage of development. An expression cassette, or fragment thereof, can also be referred to as "inserted polynucleotide" or "insertion polynucleotide" when transformed into a plant.

A "gene" is defined herein as a hereditary unit consisting of a polynucleotide that occupies a specific location on a chromosome and that contains the genetic instruction for a particular characteristic or trait in an organism.

"Genetic engineering", "transformation" and "genetic modification" are all used herein as synonyms for the transfer of isolated and cloned genes into the DNA, usually the chromosomal DNA or genome, of another organism.

The term "genotype" refers to the genetic constitution of a cell or organism. An individual's "genotype for a set of genetic markers" includes the specific alleles, for one or more genetic marker loci, present in the individual. As is known in the art, a genotype can relate to a single locus or to multiple loci, whether the loci are related or unrelated and/or are linked or unlinked. In some embodiments, an individual's genotype relates to one or more genes that are related in that the one or more of the genes are involved in the expression of a phenotype of interest (e.g., a quantitative trait as defined herein). Thus, in some embodiments a genotype comprises a sum of one or more alleles present within an individual at one or more genetic loci of a quantitative trait. In some embodiments, a genotype is expressed in terms of a haplotype (defined herein below).

The term "heterologous" when used in reference to a gene or nucleic acid refers to a gene encoding a factor that is not in its natural environment (i.e., has been altered by the hand of man). For example, a heterologous gene may include a gene from one species introduced into another species. A heterologous gene may also include a gene native to an organism that has been altered in some way (e.g., mutated, added in multiple copies, linked to a non-native promoter or enhancer polynucleotide, etc.). Heterologous genes further may comprise plant gene polynucleotides that comprise cDNA forms of a plant gene; the cDNAs may be expressed in either a sense (to produce mRNA) or anti-sense orientation (to produce an anti-sense RNA transcript that is complementary to the mRNA transcript). In one aspect of the invention, heterologous genes are distinguished from endogenous plant genes in that the heterologous gene polynucleotide are typically joined to polynucleotides comprising regulatory elements such as promoters that are not found naturally associated with the gene for the protein encoded by the heterologous gene or with plant gene polynucleotide in the chromosome, or are associated with portions of the chromosome not found in nature (e.g., genes expressed in loci where the gene is not normally expressed). Further, in embodiments, a "heterologous" polynucleotide is a polynucleotide not naturally associated with a host cell into which it is introduced, including non-naturally occurring multiple copies of a naturally occurring polynucleotide.

The terms "homology", "sequence similarity" or "sequence identity" of nucleotide or amino acid sequences mean a degree of identity or similarity of two or more sequences and may be determined conventionally by using known software or computer programs such as the Best-Fit or Gap pairwise comparison programs (GCG Wisconsin Package, Genetics Computer Group, 575 Science Drive, Madison, Wis. 53711). BestFit uses the local homology algorithm of Smith and Waterman, Advances in Applied Mathematics 2:482-489 (1981), to find the best segment of identity or similarity between two sequences. Sequence comparison between two or more polynucleotides or polypeptides is generally performed by comparing portions of the two sequences over a comparison window to identify and compare local regions of sequence similarity. The comparison window is generally from about 20 to 200 contiguous nucleotides. Gap performs global alignments: all of one sequence with all of another similar sequence using the method of Needleman and Wunsch, J. Mol. Biol. 48:443-453 (1970). When using a sequence alignment program such as BestFit to determine the degree of DNA sequence homology, similarity or identity, the default setting may be used, or an appropriate scoring matrix may be selected to optimize identity, similarity or homology scores. Similarly, when using a program such as BestFit to determine sequence identity, similarity or homology between two different amino acid sequences, the default settings may be used, or an appropriate scoring matrix, such as blosum45 or blosum80, may be selected to optimize identity, similarity or homology scores.

"Homologous recombination" is the exchange ("crossing over") of DNA fragments between two DNA molecules or chromatids of paired chromosomes in a region of identical polynucleotides. A "recombination event" is herein understood to mean a meiotic crossing-over.

The term "heterozygous" means a genetic condition existing when different alleles reside at corresponding loci on homologous chromosomes.

The term "homozygous" means a genetic condition existing when identical alleles reside at corresponding loci on homologous chromosomes.

The term "hybrid" in the context of nucleic acids refers to a double-stranded nucleic acid molecule, or duplex, formed by hydrogen bonding between complementary nucleotide bases. The terms "hybridize" or "anneal" refer to the process by which single strands of polynucleotides form double-helical segments through hydrogen bonding between complementary bases.

The term "isolated," when used in the context of the nucleic acid molecules or polynucleotides of the present invention, refers to a polynucleotide that is identified within and isolated/separated from its chromosomal polynucleotide context within the respective source organism. An isolated nucleic acid or polynucleotide is not a nucleic acid as it occurs in its natural context, if it indeed has a naturally occurring counterpart. In contrast, non-isolated nucleic acids are nucleic acids such as DNA and RNA, which are found in the state they exist in nature. For example, a given polynucleotide (e.g., a gene) is found on the host cell chromosome in proximity to neighboring genes. The isolated nucleic acid molecule may be present in single-stranded or double-stranded form. Alternatively, it may contain both the sense and antisense strands (i.e., the nucleic acid molecule may be double-stranded). In a preferred embodiment, the nucleic acid molecules of the present invention are understood to be isolated.

The term "linkage," and grammatical variants thereof, refers to the tendency of alleles at different loci on the same chromosome to segregate together more often than would be expected by chance if their transmission were independent, in some embodiments as a consequence of their physical proximity.

The phrase "linkage disequilibrium" (also called "allelic association") refers to a phenomenon wherein particular alleles at two or more loci tend to remain together in linkage groups when segregating from parents to offspring with a greater frequency than expected from their individual frequencies in a given population. For example, a genetic marker allele and a QTL allele can show linkage disequilibrium when they occur together with frequencies greater than those predicted from the individual allele frequencies. Linkage disequilibrium can occur for several reasons including, but not limited to the alleles being in close proximity on a chromosome.

The term "linkage group" refers to all of the genes or genetic traits that are located on the same chromosome. Within the linkage group, those loci that are close enough together will exhibit linkage in genetic crosses. Since the probability of crossover increases with the physical distance between genes on a chromosome, genes whose locations are far removed from each other within a linkage group may not exhibit any detectable linkage in direct genetic tests. The term "linkage group" is mostly used to refer to genetic loci that exhibit linked behavior in genetic systems where chromosomal assignments have not yet been made. Thus, in the present context, the term "linkage group" is synonymous to (the physical entity of) chromosome.

The term "locus" refers to a position (e.g., of a gene, a genetic marker, or the like) on a chromosome of a given species.

The terms "messenger RNA" or "mRNA" refer to RNA that does not comprise introns and that can be translated into a protein by the cell.

The terms "molecular marker" or "genetic marker" refer to an indicator that is used in methods for visualizing differences in characteristics of polynucleotides. It refers to a feature of an individual's genome (e.g., a polynucleotide that is present in an individual's genome) that is associated with one or more loci of interest. In some embodiments, a genetic marker is polymorphic in a population of interest or the locus occupied by the polymorphism, depending on the context. Genetic markers include, for example, single nucleotide polymorphisms (SNPs), indels (i.e., insertions/deletions), simple sequence repeats (also named microsatellite markers; SSRs), restriction fragment length polymorphisms (RFLPs), random amplified polymorphic DNAs (RAPDs), cleaved amplified polymorphic sequence (CAPS) markers, Diversity Arrays Technology (DArT) markers, and amplified fragment length polymorphisms (AFLPs), among many other examples. Additional markers include insertion mutations, sequence-characterized amplified regions (SCARs), or isozyme markers or combinations of the markers described herein which defines a specific genetic and chromosomal location. Genetic markers can, for example, be used to locate genetic loci containing alleles that contribute to variability in expression of phenotypic traits on a chromosome. The phrase "genetic marker" can also refer to the sequence of a polynucleotide complementary to a genomic polynucleotide, such as a sequence of a nucleic acid used as a probe. A genetic marker can be physically located in a position on a chromosome that is within or outside of the genetic locus with which it is associated (i.e., is intragenic or extragenic, respectively). Stated another way, whereas genetic markers are typically employed when the location on a chromosome of the gene that corresponds to the locus of interest has not been identified and there is a non-zero rate of recombination between the genetic marker and the locus of interest, the presently disclosed subject matter can also employ genetic markers that are physically within the boundaries of a genetic locus (e.g., inside a genomic polynucleotide that corresponds to a gene such as, but not limited to a polymorphism within an intron or an exon of a gene).

The term "microsatellite or SSRs (simple sequence repeats) marker" is understood within the scope of the invention to refer to a type of genetic marker that comprises numerous repeats of short sequences of DNA bases, which are found at loci throughout the plant's DNA and have a likelihood of being highly polymorphic.

The phrase "nucleic acid" or "polynucleotide" refers to any physical string of monomer units that can be corresponded to a string of nucleotides, including a polymer of nucleotides (e.g., a typical DNA polymer or poly deoxyribonucleotide or RNA polymer or polyribonucleotide), modified oligonucleotides (e.g., oligonucleotides comprising bases that are not typical to biological RNA or DNA, such as 2'-O-methylated oligonucleotides), and the like. In some embodiments, a nucleic acid or polynucleotide can be single-stranded, double-stranded, multi-stranded, or combinations thereof. Unless otherwise indicated, a particular nucleic acid or polynucleotide of the present invention optionally comprises or encodes complementary polynucleotides, in addition to any polynucleotide explicitly indicated.

"Operably linked" refers to the association of polynucleotides on a single nucleic acid fragment so that the function of one affects the function of the other. For example, a promoter is operably linked with a coding polynucleotide or functional RNA when it is capable of affecting the expression of that coding polynucleotide or functional RNA (i.e., that the coding polynucleotide or functional RNA is under the transcriptional control of the promoter). Coding polynucleotide in sense or antisense orientation can be operably linked to regulatory polynucleotides.

"PCR (polymerase chain reaction)" is understood within the scope of the invention to refer to a method of producing relatively large amounts of specific regions of DNA, thereby making possible various analyses that are based on those regions.

"Polymorphism" is understood within the scope of the invention to refer to the presence in a population of two or more different forms of a gene, genetic marker, or inherited trait.

The term "probe" refers to a single-stranded oligonucleotide that will form a hydrogen-bonded duplex with a substantially complementary oligonucleotide in a target nucleic acid analyte or its cDNA derivative.

The term "primer", as used herein, refers to an oligonucleotide which is capable of annealing to the amplification target allowing a DNA polymerase to attach, thereby serving as a point of initiation of DNA synthesis when placed under conditions in which synthesis of primer extension product is induced, e.g., in the presence of nucleotides and an agent for polymerization such as DNA polymerase and at a suitable temperature and pH. The (amplification) primer is preferably single stranded for maximum efficiency in amplification. Preferably, the primer is an oligodeoxyribonucleotide. The primer is generally sufficiently long to prime the synthesis of extension products in the presence of the agent for polymerization. The exact lengths of the primers will depend on many factors, including temperature and composition (A/T and G/C content) of primer. A pair of bi-directional primers consists of one forward and one reverse primer as commonly used in the art of DNA amplification such as in PCR amplification. It will be understood that "primer," as used herein, may refer to more than one primer, particularly in the case where there is some ambiguity in the information regarding the terminal sequence(s) of the target region to be amplified. Hence, a "primer" includes a collection of primer oligonucleotides containing sequences representing the possible variations in the sequence or includes nucleotides which allow a typical base pairing. The oligonucleotide primers may be prepared by any suitable method. Methods for preparing oligonucleotides of specific sequence are known in the art, and include, for example, cloning and restriction of appropriate sequences, and direct chemical synthesis. Chemical synthesis methods may include, for example, the phospho di- or tri-ester method, the diethylphosphoramidate method and the solid support method disclosed in, for example, US 4,458,066. The primers may be labeled, if desired, by incorporating means detectable by, for instance, spectroscopic, fluorescence, photochemical, biochemical, immunochemical, or chemical means. Template-dependent extension of the oligonucleotide primer(s) is catalyzed by a polymerizing agent in the presence of adequate amounts of the four deoxyribonucleotide triphosphates (dATP, dGTP, dCTP and dTTP, i.e. dNTPs) or analogues, in a reaction medium which is comprised of the appropriate salts, metal cations, and pH buffering system. Suitable polymerizing agents are enzymes known to catalyze primer- and template-dependent DNA synthesis. Known DNA polymerases include, for example, E. coli DNA polymerase I or its Klenow fragment, T4 DNA polymerase, and Taq DNA polymerase. The reaction conditions for catalyzing DNA synthesis with these DNA polymerases are known in the art. The products of the synthesis are duplex molecules consisting of the template strands and the primer extension strands, which include the target sequence. These products, in turn, serve as template for another round of replication. In the second round of replication, the primer extension strand of the first cycle is annealed with its complementary primer; synthesis yields a "short" product which is bound on both the 5'- and the 3'-ends by primer sequences or their complements. Repeated cycles of denaturation, primer annealing, and extension result in the exponential accumulation of the target region defined by the primers. Sufficient cycles are run to achieve the desired amount of polynucleotide containing the target region of nucleic acid. The desired amount may vary, and is determined by the function which the product polynucleotide is to serve. The PCR method is well described in handbooks and known to the skilled person. After amplification by PCR, the target polynucleotides may be detected by hybridization with a probe polynucleotide which forms a stable hybrid with that of the target sequence under low, moderate or even highly stringent hybridization and wash conditions. If it is expected that the probes will be essentially completely complementary (i.e., about 99% or greater) to the target sequence, highly stringent conditions may be used. If some mismatching is expected, for example if variant strains are expected with the result that the probe will not be completely complementary, the stringency of hybridization may be lessened. However, conditions are typically chosen which rule out nonspecific/adventitious binding. Conditions, which affect hybridization, and which select against nonspecific binding are known in the art, and are described in, for example, Sambrook and Russell, 2001. Generally, lower salt concentration and higher temperature increase the stringency of hybridization conditions. "PCR primer" is preferably understood within the scope of the present invention to refer to relatively short fragments of single-stranded DNA used in the PCR amplification of specific regions of DNA.

The terms "protein," "peptide" and "polypeptide" are used interchangeably herein.

The term "promoter" refers to a polynucleotide, usually upstream (5') of its coding polynucleotide, which controls the expression of the coding polynucleotide by providing the recognition for RNA polymerase and other factors required for proper transcription.

"Constitutive promoter" refers to a promoter that is able to express the open reading frame (ORF) that it controls in all or nearly all of the plant tissues during all or nearly all developmental stages of the plant (referred to as "constitutive expression"). "Regulated promoter" refers to promoters that direct gene expression not constitutively, but in a temporally- and/or spatially-regulated manner, and includes tissue-specific, tissue-preferred and inducible promoters. It includes natural and synthetic polynucleotides as well as polynucleotides which may be a combination of synthetic and natural polynucleotides. Different promoters may direct the expression of a gene in different tissues or cell types, or at different stages of development, or in response to different environmental conditions.

As used herein, gene or trait "stacking" is combining desired genes or traits into one transgenic plant line. As one approach, plant breeders stack transgenic traits by making crosses between parents that each have a desired trait and then identifying offspring that have both of these desired traits (so-called "breeding stacks"). Another way to stack genes is by transferring two or more genes into the cell nucleus of a plant at the same time during transformation. Another way to stack genes is by re-transforming a transgenic plant with another gene of interest. For example, gene stacking can be used to combine two different insect resistance traits, an insect resistance trait and a disease resistance trait, or a herbicide resistance trait (such as, for example, Bt11). The use of a selectable marker in addition to a gene of interest would also be considered gene stacking.

"Tissue-specific promoter" or "tissue-preferred promoter" refers to regulated promoters that are not expressed in all plant cells but only or preferentially in one or more cell types in specific organs (such as leaves or seeds), specific tissues (such as embryo or cotyledon), or specific cell types (such as leaf parenchyma or seed storage cells). These terms also include promoters that are temporally regulated, such as in early or late embryogenesis, during fruit ripening in developing seeds or fruit, in fully differentiated leaf, or at the onset of senescence. Those skilled in the art will understand that tissue-specific promoters need not exhibit an absolute tissue-specificity, but mediate transcriptional activation in most plant parts at a level of about 1% or less of the level reached in the part of the plant in which transcription is most active.

"Inducible promoter" refers to those regulated promoters that can be turned on in one or more cell types by an external stimulus, such as a chemical, light, hormone, stress, or a pathogen.

The terms "stringent conditions" or "stringent hybridization conditions" include reference to conditions under which a polynucleotide will hybridize to its target sequence to a detectably greater degree than other sequences (e.g., at least 2-fold over background). Stringent conditions are sequence-dependent and will be different in different circumstances. By controlling the stringency of the hybridization and/or washing conditions, target polynucleotides can be identified which are 100% complementary to the probe (homologous probing). Alternatively, stringency conditions can be adjusted to allow some mismatching in sequences so that lower degrees of similarity are detected (heterologous probing). Typically, stringent conditions will be those in which the salt concentration is less than approximately 1.5 M Na ion, typically about 0.01 to 1.0 M Na ion (or other salts) at pH 7.0 to 8.3 and the temperature is at least about 30° C for short probes (e.g., 10 to 50 nucleotides) and at least about 60° C for long probes (e.g., greater than 50 nucleotides). Stringent conditions also may be achieved with the addition of destabilizing agents such as formamide. Exemplary low stringency conditions include hybridization with a buffer solution of 30 to 35% formamide, 1 M NaCl, 1% SDS (w/v; sodium dodecyl sulphate) at 37° C, and a wash in 1 × to 2×SSC (20×SSC = 3.0 M NaCl/0.3 M trisodium citrate) at 50 to 55° C. Exemplary moderate stringency conditions include hybridization in 40 to 45% formamide, 1 M NaCl, 1% SDS at 37° C, and a wash in 0.5× to 1×SSC at 55 to 60° C. Exemplary high stringency conditions include hybridization in 50% formamide, 1 M NaCl, 1% SDS at 37°C, and a wash in 0.1×SSC at 60 to 65° C. Specificity is typically the function of post-hybridization washes, the critical factors being the ionic strength and temperature of the final wash solution. For DNA-DNA hybrids, the Tm can be approximated from the equation of Meinkoth and Wahl (Anal. Biochem., 138:267-284, 1984): Tm=81.5° C+16.6 (log M)+0.41 (% GC)-0.61 (% form)-500/L; where M is the molarity of monovalent cations, % GC is the percentage of guanosine and cytosine nucleotides in the DNA, % form is the percentage of formamide in the hybridization solution, and L is the length of the hybrid in base pairs. The Tm is the temperature (under defined ionic strength and pH) at which 50% of a complementary target sequence hybridizes to a perfectly matched probe. Tm is reduced by about 1° C for each 1% of mismatching; thus, Tm, hybridization and/or wash conditions can be adjusted to hybridize to sequences of the desired identity. For example, if sequences with approximately 90% identity are sought, the Tm can be decreased 10° C. Generally, stringent conditions are selected to be about 5° C lower than the thermal melting point (Tm) for the specific sequence and its complement at a defined ionic strength and pH. However, severely stringent conditions can utilize hybridization and/or wash at 1, 2, 3, or 4° C lower than the thermal melting point (Tm); moderately stringent conditions can utilize a hybridization and/or wash at 6, 7, 8, 9, or 10° C lower than the thermal melting point (Tm); low stringency conditions can utilize a hybridization and/or wash at 11, 12, 13, 14, 15, or 20° C lower than the thermal melting point (Tm). Using the equation, hybridization and wash compositions, and desired Tm, those of ordinary skill will understand that variations in the stringency of hybridization and/or wash solutions are inherently described. If the desired degree of mismatching results in a Tm of less than 45° C (aqueous solution) or 32° C (formamide solution), it is preferred to increase the SSC concentration so that a higher temperature can be used. An extensive guide to the hybridization of nucleic acids is found in Tijssen, Laboratory Techniques in Biochemistry and Molecular Biology - Hybridization with Nucleic Acid Probes, Part I, Chapter 2 "Overview of principles of hybridization and the strategy of nucleic acid probe assays", Elsevier, N.Y. (1993); and Current Protocols in Molecular Biology, Chapter 2, Ausubel, et al., eds., Greene Publishing and Wiley-Interscience, New York (1995). Methods of stringent hybridization are known in the art which conditions can be calculated by means known in the art. This is disclosed in Sambrook et al., Molecular Cloning: A Laboratory Manual, 2nd Ed., Cold Spring Harbor Laboratory Press, 1989, Cold Spring Harbor, N.Y. and Current Protocols in Molecular Biology, Ausebel et al, eds., John Wiley and Sons, Inc., 2000. Methods of determining percent sequence identity are known in the art, an example of which is the GCG computer sequence analysis software (GCG, Inc, Madison Wis.).

The term "tilling" (or "TILLING" for "Targeting-Induced Local Lesions IN Genomes") refers to a non-transgenic approach for the generation of null or knockout alleles of a certain gene of interest (MCCALLUM et al., 2000, Plant Physiology 123:439-442). A "transgene" refers to a gene, polynucleotide or nucleic acid introduced into the genome of an organism by genetic manipulation in order to alter its genotype. Transgenes may include, for example, genes, polynucleotides or nucleic acids that are either heterologous or homologous to the particular plant to be transformed. Additionally, transgenes may comprise native genes inserted into a non-native organism, or chimeric genes, polynucleotides or nucleic acids

### DETAILED DESCRIPTION

In one embodiment, the invention is drawn to a method of preserving a tissue sample containing nucleic acids for use in DNA analysis, comprising (a) obtaining a tissue sample; and (b) contacting the tissue sample with an alkaline glycerol solution at ambient temperature, as defined in appended claim 1. In another embodiment, the alkaline glycerol solution is a reagent for processing a biological sample containing nucleic acids for use in analysis, comprising an amount of alkali and a glycerol capable of effectively lysing the sample, releasing the nucleic acids, inactivating PCR inhibitors, and preserving the nucleic acids at ambient temperatures for use in analysis, as defined in appended claim 2. As used herein, "ambient temperatures" means temperatures between approximately 4.4 °C and 35 °C (40 °F to 95 °F), inclusive, or any temperature below boiling. In one embodiment, the reagent is at a temperature between -20°C to 45°C, inclusive. The alkali is capable of maintaining the pH of the reagent from between pH 12 to pH 13.8, inclusive. In one preferred aspect, the alkali is sodium hydroxide (NaOH). The glycerol is at a concentration from 10% to 25%, and preferably at a concentration at 15% or 20%. The reagent further comprises a detergent which is polyvinyl pyrrolidone (PVP).

In another embodiment, the invention provides a kit for storage of tissue samples containing nucleic acids. According to the claimed invention, the kit includes the alkali glycerol reagent of the invention. In another aspect, the kit provides a storage vessel. In one aspect, the storage vessel is an Eppendorf tube or series of tubes; in another aspect, the storage vessel is a 96-well block capable of holding the tissue sample and the reagent.

In another embodiment, the invention provides a method of extracting nucleic acids, comprising: (a) obtaining a tissue sample containing nucleic acids; (b) contacting the tissue sample with the reagent of claim 2; and (c) disrupting the tissue sample; wherein the nucleic acids are released from the tissue sample and the nucleic acids are suitable for immediate analysis or for storage and preservation. In another embodiment, the invention provides for performing genotyping (or qPCR) on preserved nucleic acids comprising obtaining extracted nucleic acids by the method of extracting nucleic acids and subjecting the extracted nucleic acids to genotyping (or qPCR).

These embodiments and aspects of the invention may be more readily understood by way of the examples, below.

### EXAMPLES

### I. Alkali Effect on DNA Yield

Maize leaf discs approximately 6mm wide (about the size of a paper hole punch) were collected; one leaf disc per well of a 96-well block. In some instances, four leaf discs approximately 3mm wide were collected. Approximately 0.1 mL of alkali containing solution was added to each well. A variety of alkaline concentrations was used among other components unchanged: from 10 mM sodium hydroxide (NaOH) to 200 mM NaOH. The sample block was sealed and mixed for a few times manually. After usual sample shipping and storage at different ambient temperatures in 1 day, e.g. 37°C for about 3 hours and room temperature for about 15 hours, the leaf tissues in alkali were ground with metal beads. The pH of alkaline cell lysate was reduced by adding equal amount of Tris-HCl buffer, pH7.5. The DNA released from ground leaf tissue in supernatant (upper liquid) after centrifugation (insoluble pellet on bottom) was used in DNA analysis, e.g. PCR, with appropriate dilution.

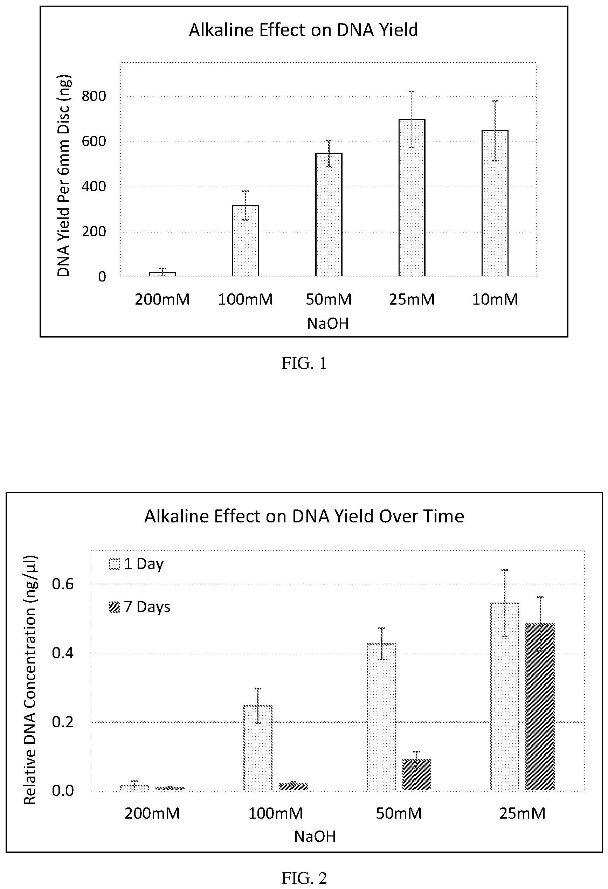

The effect of alkali on DNA yield from plant tissue samples was measured after one day incubation and one week incubation. Relative DNA yield was measured by quantitative PCR (qPCR) analysis with a TaqMan assay targeting an endogenous gene, in terms of "cycle threshold" (Ct). A lower Ct indicates a higher yield of DNA. By starting with the Ct, the total DNA yield based on the amount of DNA present in qPCR can be calculated using the following formula: relative DNA concentration to a standard reference DNA control x volume of the supernatant x dilution factor. *See, e.g.,* www.csun.edu/~hcbio027/biotechnology/lec3/pcr/p.htm. See Table 1 for total DNA yield per leaf disc after 1 day incubation and Table 2 for relative DNA yield represented by DNA concentration (ng/µl) in qPCR over different duration of incubation and DNA in consistent dilution, which correspond to Figs. 1 and 2, respectively.

**Table 1. NaOH effect on DNA yield after incubation for 1 day (15 hours at room temperature plus 3 hours at 37°C).**

| NaOH Concentration | TaqMan qPCR | | Relative DNA Yield | |
|---|---|---|---|---|
| | (Ct) | SD | (ng) | SD |
| 200mM | 31.7 | 1.2 | 20.1 | 16.8 |
| 100mM | 27.4 | 0.3 | 316.2 | 64.1 |
| 50mM | 26.6 | 0.2 | 547.2 | 58.6 |
| 25mM | 26.2 | 0.3 | 698.0 | 123.9 |
| 10mM | 26.7 | 0.3 | 648.2 | 132.5 |

**Table 2. NaOH effect on DNA yield after incubation for 1 day and 1 week. 1 day is 15 hours at room temperature plus 3 hours at 37°C. 1 week includes five days at room temperature plus 40 hours at 37°C.**

| NaOH Concentration | 1-Day Yield | | 1-Week Yield | |
|---|---|---|---|---|
| | (ng/ul) | SD | (ng/µl) | SD |
| 200mM | 0.02 | 0.01 | 0.01 | 0.00 |
| 100mM | 0.25 | 0.05 | 0.02 | 0.01 |
| 50mM | 0.43 | 0.05 | 0.09 | 0.02 |
| 25mM | 0.55 | 0.10 | 0.48 | 0.08 |

### II. Glycerol effect on DNA yield.

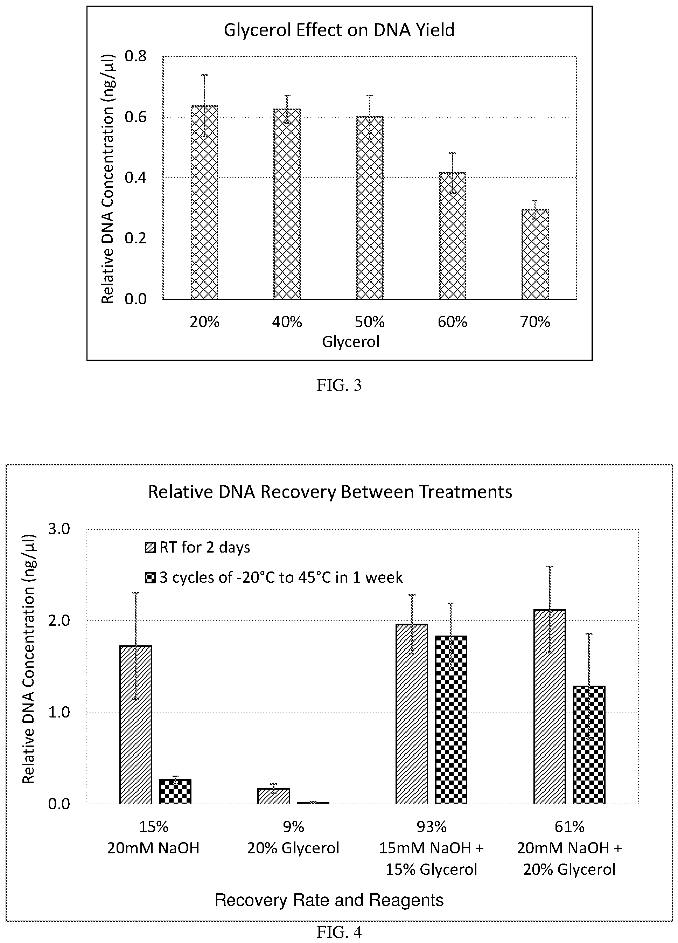

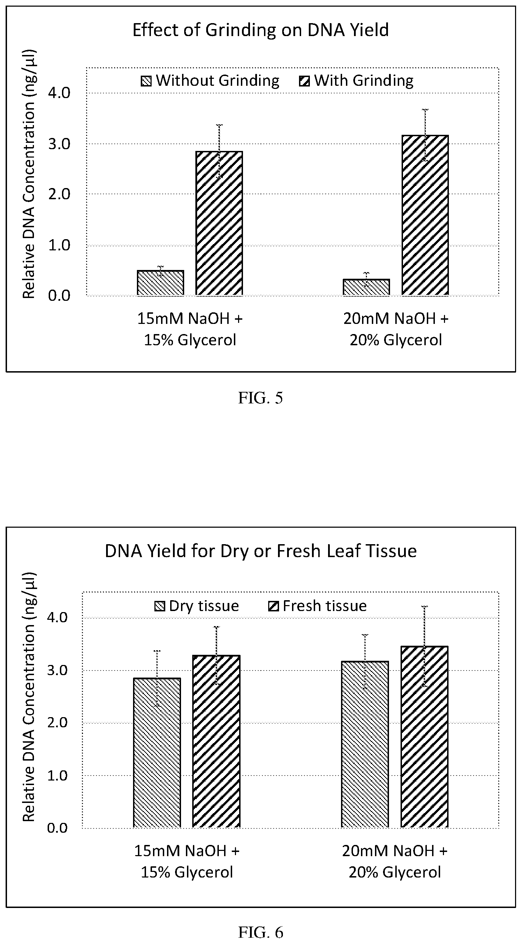

Similarly, the effect of glycerol on DNA yield from plant tissue samples was measured after one day incubation. Relative DNA yield was measured by qPCR analysis with a TaqMan assay targeting an endogenous gene. A variety of glycerol percentages (%) in weight/volume (w/v) was used among other components unchanged: from 70% down to 20%. The leaf samples were processed within 1 day of incubation. The resulting DNA in supernatant after centrifugation was used in qPCR analysis, with appropriate dilution. See Table 3 for relative DNA yield indicated by DNA concentration (with consistent DNA dilution for qPCR) which were calculated based on Ct values for samples and reference control. The corresponding figure is Fig. 3.

**Table 3. Glycerol effect on DNA yield after incubation in 1 day.**

| Glycerol (w/v) | Yield (ng/µl) | SD |
|---|---|---|
| 20% | 0.64 | 0.10 |
| 40% | 0.63 | 0.04 |
| 50% | 0.60 | 0.07 |
| 60% | 0.42 | 0.07 |
| 70% | 0.30 | 0.03 |

### III. Relative DNA recovery between short and long incubations under different treatments

A key purpose for preserving leaf tissue DNA in common sampling environments over a period of time is to recover adequate DNA with suitable quality for DNA analysis such as genotyping and PCR. In order to achieve best DNA yield under various conditions simulated with different treatments, a variety of chemicals and chemical combinations had been tested for leaf tissue preservation and DNA extraction in one process, with goals of high DNA concentration (and so recovery) and high quality for PCR. DNA yields and relative recovery rates after longer treatments were determined by qPCR analysis. Details of the selected chemicals and chemical combinations in this example can be found in Table 4 under Treatment. 2-Day incubation was at room temperature and DNA yield for each treatment serves as control (set as 100%) for comparison. Recovery rates were calculated after longer incubation which includes 3 cycles of -20°C for overnight and 45°C for 8 hours in 1 week. The recovery rates in percentage for each pair of incubations were also demonstrated in Fig. 4.

**Table 4.**

| Treatment | 2-Day Yield | | 1-Week Yield | | Recovery |
|---|---|---|---|---|---|
| | (ng/µl) | SD | (ng/µl) | SD | |
| 20mM NaOH only | 1.73 | 0.58 | 0.27 | 0.04 | 15.3% |
| 20% Glycerol only | 0.17 | 0.05 | 0.02 | 0.01 | 9.0% |
| 15mM NaOH + 15% Glycerol | 1.96 | 0.32 | 1.83 | 0.36 | 93.3% |
| 20mM NaOH + 20% Glycerol | 2.12 | 0.47 | 1.29 | 0.57 | 60.1% |

### IV. DNA concentration, quality and stability

Surprisingly, the chemical combination of alkali and glycerol at low concentrations produced high DNA yield and more importantly, better DNA recovery than other chemicals or chemical combinations tested in this and other experiments over longer period of time at various temperatures. Other chemicals and chemical combinations used in literatures and existing methods were included in comparisons for leaf DNA preservation and DNA extraction under similar treatments. None of them had comparable DNA recovery rate to the chemical combination of alkaline glycerol discovered in this new method for preservation of tissue DNA and simple DNA extraction (results not shown).

A well-known chemical PVP was added in to the alkaline glycerol to inactivate polyphenols which are rich in plant materials and often cause significant DNA damage. The concentration of PVP was in range of 0.5% to 3%, preferably in 1% for majority of tissues. The preferred chemical combination-alkaline glycerol plus PVP-is able to protect tissue DNA from damage under various temperatures, especially at high ambient temperatures, and for an extended period such as a few months before and after tissue sample processing. So it provides a new approach for simple leaf DNA preservation and easy DNA extraction in a single process. The DNA is in good quality suitable for direct PCR analysis without purification and in adequate concentration for most DNA analysis. For example: the DNA made from a single leaf disc with the preferred reagent was good for genotyping and qPCR analyses without dilution (of the DNA) and with dilution until after 27-fold dilution. The DNA is also stable for months at least at room temperatures.

Advantages of the preferred chemical reagent-alkaline glycerol plus PVP-allow to easily achieve top DNA yield with limited tissue input. The simple process also allows to achieve very high throughput for sample processing at production scale. It can also reduce running costs including consumables and hands-on time needed.

### V. Effect of Grinding on DNA yield

Some tough tissues or fungal spores may need grinding step for efficient DNA release from cells and so for high DNA yield or concentration. This example is for DNA extraction from a dry leaf disc with treatments (see Table 5) and incubation for overnight at room temperature. Grinding was at above 900rpm for 4 minutes with steel beads. The DNA yield was significantly higher with grinding than without grinding. In fact, mechanic grinding is an efficient method for tissue disruption in terms of throughput and costs. Some cultured cells may not need this grinding step. The DNA concentration without grinding is low but maybe enough for genotyping analysis and presence/absence analysis by PCR. Higher DNA concentration is typically better for qPCR analysis.

**Table 5.**

| Treatment | DNA Yield without Grinding | | DNA Yield with Grinding | |
|---|---|---|---|---|
| | (ng/µl) | SD | (ng/µl) | SD |
| 15mM NaOH + 15% Glycerol | 0.49 | 0.09 | 2.85 | 0.52 |
| 20mM NaOH + 20% Glycerol | 0.32 | 0.13 | 3.17 | 0.51 |

### VI. DNA yield comparisons between fresh and dry tissue

DNA yield was usually higher starting with fresh tissue than with dry tissue. However, handling fresh or frozen tissue requires more resources. This new DNA extraction method with preferred reagent was also suitable to take dry tissue as alternative starting material. This example was to compare the DNA yield between dry and fresh leaf tissue. With same treatments and similar leaf tissue size the relative DNA yield for dry leaf tissue was about 80%-90% compared to the yield for fresh leaf tissue. The DNA recovery from dry leaf tissue with this new method was fairly high.

**Table 6.**

| Treatment | Yield for Dry Tissue | | Yield for Fresh Tissue | | Dry/Fresh |
|---|---|---|---|---|---|
| | (ng/µl) | SD | (ng/µl) | SD | |
| 15mM NaOH + 15% Glycerol | 2.85 | 0.52 | 3.29 | 0.54 | 86.8% |
| 20mM NaOH + 20% Glycerol | 3.17 | 0.51 | 3.46 | 0.76 | 91.7% |

## Claims

1. A method of preserving a tissue sample containing nucleic acids for use in DNA analysis, comprising:
a. obtaining a tissue sample; and
b. contacting the tissue sample with an alkaline glycerol solution at an ambient temperature, thereby preserving the nucleic acids for an extended period at ambient temperatures for use in DNA analysis
wherein the alkaline glycerol solution comprises an amount of alkali, glycerol, and detergent, wherein the alkali is capable of maintaining the pH of the reagent from pH 12 to pH 13.8, wherein the glycerol is at a concentration from 10% to 25%, and wherein the detergent is polyvinyl pyrrolidone (PVP).

2. A reagent for processing a biological sample containing nucleic acids for use in analysis, comprising an amount of alkali, glycerol, and detergent capable of effectively lysing the sample, releasing the nucleic acids, inactivating PCR inhibitors, and preserving the nucleic acids for an extended period at ambient temperatures for use in analysis, wherein the alkali is capable of maintaining the pH of the reagent from pH 12 to pH 13.8, wherein the glycerol is at a concentration from 10% to 25%, and wherein the detergent is PVP.

3. The reagent of claim 2, wherein the alkali is NaOH.

4. The reagent of claim 2, wherein the glycerol is at a concentration at 15% or 20%.

5. A kit for storage of tissue samples containing nucleic acids, comprising the reagent of claim 2.

6. A method of extracting nucleic acids comprising:
a. obtaining a tissue sample containing nucleic acids;
b. contacting the tissue sample with the reagent of claim 2; and
c. disrupting the tissue sample;
wherein the nucleic acids are released from the tissue sample and the nucleic acids are suitable for immediate analysis or for storage and preservation.

## Patentansprüche

1. Verfahren zur Konservierung einer Nukleinsäuren enthaltenden Gewebeprobe zur Verwendung bei der DNA-Analyse, umfassend:
a. Gewinnen einer Gewebeprobe; und
b. In-Kontakt-Bringen der Gewebeprobe mit einer alkalischen Glycerinlösung bei einer Umgebungstemperatur, wodurch die Nukleinsäuren über einen längeren Zeitraum bei Umgebungstemperaturen zur Verwendung bei der DNA-Analyse konserviert werden, wobei die alkalische Glycerinlösung eine Menge an Alkali, Glycerin und Detergens umfasst, wobei durch das Alkali der pH des Reagens auf pH 12 bis pH 13,8 gehalten werden kann, wobei das Glycerin in einer Konzentration von 10 % bis 25 % vorliegt und wobei es sich bei dem Detergens um Polyvinylpyrrolidon (PVP) handelt.

2. Reagens zur Verarbeitung einer Nukleinsäuren enthaltenden biologischen Probe zur Verwendung bei der Analyse, umfassend eine Menge an Alkali, Glycerin und Detergens, durch die wirksam die Probe lysiert werden kann, die Nukleinsäuren freigesetzt, PCR-Inhibitoren inaktiviert und die Nukleinsäuren über einen längeren Zeitraum bei Umgebungstemperaturen zur Verwendung bei der Analyse konserviert werden können, wobei durch das Alkali der pH des Reagens auf pH 12 bis pH 13,8 gehalten werden kann, wobei das Glycerin in einer Konzentration von 10 % bis 25 % vorliegt und wobei es sich bei dem Detergens um PVP handelt.

3. Reagens nach Anspruch 2, wobei es sich bei dem Alkali um NaOH handelt.

4. Reagens nach Anspruch 2, wobei das Glycerin in einer Konzentration bei 15 % oder 20 % vorliegt.

5. Kit zur Lagerung von Nukleinsäuren enthaltenden Gewebeproben, umfassend das Reagens nach Anspruch 2.

6. Verfahren zur Extraktion von Nukleinsäuren, umfassend:
a. Gewinnen einer Nukleinsäuren enthaltenden Gewebeprobe;
b. In-Kontakt-Bringen der Gewebeprobe mit dem Reagens nach Anspruch 2; und
c. Aufschließen der Gewebeprobe;
wobei die Nukleinsäuren aus der Gewebeprobe freigesetzt werden und für die sofortige Analyse oder für Lagerung und Konservierung geeignet sind.

## Revendications

1. Procédé de préservation d'un échantillon de tissu contenant des acides nucléiques pour une utilisation dans l'analyse d'ADN, comprenant :
a. l'obtention d'un échantillon de tissu ; et
b. la mise en contact de l'échantillon de tissu avec une solution alcaline de glycérol à une température ambiante, préservant ainsi les acides nucléiques pendant une période prolongée à des températures ambiantes pour une utilisation dans l'analyse d'ADN
la solution alcaline de glycérol comprenant une quantité d'alcali, de glycérol et de détergent, l'alcali étant capable de maintenir le pH du réactif de pH 12 à pH 13,8, le glycérol étant à une concentration de 10 % à 25 %, et le détergent étant une polyvinylpyrrolidone (PVP).

2. Réactif pour le traitement d'un échantillon biologique contenant des acides nucléiques destinés pour une utilisation dans une analyse, comprenant une quantité d'alcali, de glycérol et de détergent capable de lyser efficacement l'échantillon, de libérer les acides nucléiques, d'inactiver les inhibiteurs de PCR et de préserver les acides nucléiques pendant une période prolongée à des températures ambiantes pour une utilisation dans une analyse, l'alcali étant capable de maintenir le pH du réactif de pH 12 à pH 13,8, le glycérol étant à une concentration de 10 % à 25 %, et le détergent étant une PVP.

3. Réactif selon la revendication 2, dans lequel l'alcali est NaOH.

4. Réactif selon la revendication 2, dans lequel le glycérol est à une concentration de 15 % ou 20 %.

5. Kit pour le stockage d'échantillons de tissus contenant des acides nucléiques, comprenant le réactif selon la revendication 2.

6. Procédé d'extraction d'acides nucléiques comprenant :
a. l'obtention d'un échantillon de tissu contenant des acides nucléiques ;
b. la mise en contact de l'échantillon de tissu avec le réactif selon la revendication 2 ; et
c. la perturbation de l'échantillon de tissu ;
les acides nucléiques étant libérés de l'échantillon de tissu et les acides nucléiques étant adaptés pour une analyse immédiate ou pour un stockage et une préservation.
